# EUROPEAN PATENT SPECIFICATION

(11) **EP 1 221 947 B1**
(45) Date of publication and mention of the grant of the patent: **08.12.2004**
(21) Application number: 00992193.3
(22) Date of filing: 25.08.2000
(51) Int. Cl.: A61K 9/52, A61K 31/4439

(54) **SOFT GEL CAPSULE RESISTANT TO GASTRIC JUICES**
MAGENSAFTRESISTENTE GELARTIGE WEICHKAPSEL
CAPSULE DE GELATINE MOLLE RESISTANT AU SUC GASTRIQUE

(30) Priority: 01.10.1999 IN MA096899
(43) Date of publication of application: 17.07.2002
(73) Proprietor: Natco Pharma Limited, Hyderabad 500 033 (IN)
(72) Inventor: VENKATESWARA RAO, Pavuluri, Manager R&D(F), Hyderabad 500 033 (IN); KHADGAPATHI, Podili, Director - Technical, Hyderabad 500 033 (IN)
(74) Representative: Nachshen, Neil Jacob
(86) International application number: PCT/IN2000/000079
(87) International publication number: WO 2001/024780

(56) References cited:
- EP-A- 0 960 620
- WO-A-98/50019
- DE-A- 3 222 476
- DATABASE CHEMABS [Online] CHEMICAL ABSTRACTS SERVICE, COLUMBUS, OHIO, US; LEE, SEUNG-JIN ET AL: "Omeprazole enteric-coated soft capsules" retrieved from STN Database accession no. 133:242640 CA XP002164221 & KR 131 375 B (S. KOREA) 17 April 1998 (1998-04-17)

## Description

The present invention relates to an improved pharmaceutical composition and a process for its preparation. The present invention particularly relates to an improved pharmaceutical composition, in the form of a soft gel capsule resistant to digestive juice. The composition of the present invention is made up of gelatin and an enteric polymer in the form of free acid or its salt, containing a benzimidazole derivative used in the treatment of duodenal ulcers, solublised and/or suspended in a liquid or semisolid medium, comprising of a hydrophobic carrier, an alkaline inert reacting material and a surface active agent and/or a solublising agent. The present invention also relates to a method for preparing the above said pharmaceutical composition.

Benzimidazole derivatives such as Omeprazole, Lansoprazole Timoprazole and Pantoprazole etc., are known potent proton pump inhibitors with powerful inhibitory action against the secretion of gastric juice (Lancet, Nov. 27, 1982 pages 1223-1224). They are used in the treatment of Zollinzer - Elision syndrome and stress related esophagitis ulceration. The derivatives are well known and are described, for example in EP-A 0005129.

It has been found that these benzimidazole derivatives, and in particular omeprazole, are susceptible to degradation in acid and neutral media. It is known to protect oral dosage forms of such benzimidazole derivatives by providing an enteric coating. In this way, the active material is protected from acidic gastric juices until it reaches the desired site of release, e.g. the small intestine. Because certain enteric coatings themselves can be, or contain, acidic material, it also often is required to protect the benzimidazole derivatives from the acidity of the enteric coating. For example, it is known to formulate the benzimidazole derivatives with an alkaline material before applying the enteric coating. It is also known to provide an intermediate coating between the benzimidazole derivative and the enteric coating. Generally the intermediate coating is selected so as to be substantially water-soluble or water-dispersible.

EP-A-024 7983; US 4,786,505; US 4,853,230 and US 5,385,739 describe oral pharmaceutical preparations containing benzimidazole derivatives that are potent inhibitors of gastric acid secretion, which are composed of a core material in the form of small beads or tablets containing one of the benzimidazole derivatives, particularly omeprazole, together with an alkaline reacting compound. The core material contains one or more inert reacting sub-coating layers thereon thereby providing a final outer enteric coating. Although the above-described compositions are reasonably stable over an extended period of storage, discoloration of the pellets and / or tablets with reduced gastric resistance and reduction of dissolution rate in alkaline buffers was observed.

Moreover the processes disclosed above are time-consuming and laborious, involving many stages in manufacturing of the composition, consequently increasing the cost of the final composition.

In a German patent DE 32 22 476 a pharmaceutical composition has been described in which a soft gelatin capsule that is resistant to digestive juice, whose wall includes a usual gelatin mass which contains polyvinyl acetate phthalate, hydroxypropyl methyl cellulose phthalate or a vinyl acetate /crotonic acid copolymer and/or an alkali metal salt, ammonia salt or amino salt of the same in their wall, and which released its contents readily in the intestines within the prescribed time. The capsules are further treated on the surface with an aldehyde-coating agent.

With the capsule shell composition described in DE 32 22 476 above, if used as such for manufacturing capsules containing one of the benzimidazole derivatives in a conventional manner, the free acidic groups of the polymer in the shell composition reacts with the benzimidazole derivatives and reduces the efficacy of the product during its storage / shelf life period.

The above said prior art processes also have the following drawbacks: -

Requirement of sophisticated coating equipment and large amounts of organic solvents / alkali salts are employed to dissolve the enteric polymers for coating the fine particles.

The active substance(s), benzimidazole derivatives, needs to be protected by a sub coat from the reacting acidic groups present in the enteric polymers.

The processing time and the number of steps involved are many.

The resulting product, i.e., pellets / beads / tablets, has to be dried to keep moisture content below 1.5% to ensure drug stability during processing and through its shelf storage.

The active substance(s), benzimidazole derivatives, present in the final formulation as solid dispersed in a hydrophilic solid matrix and hence requires some time to dissolve into the surrounding intestinal fluid before being absorbed.

Large quantities of polymer i.e. 15-25% w/w, based on product, need to be applied to achieve desired gastric protection.

The pH of medium used to suspend / solublise the drug needs to be adjusted to alkaline condition i.e. above pH 8.0 to prevent degradation during processing.

The micro environment surrounding the core also contains alkaline material to neutralise the acidic medium that permeates the outer enteric coating during the product transit through stomach.

In case of pellets / beads large surface area needs to be coated with protective polymer sub-coat.

Considering the importance gained for the composition containing benzimidazole derivatives, particularly for the treatment of duodenal ulcers, there is a need for the development of pharmaceutical composition containing said derivatives having stability for an extended period during which period the composition does not get discoloured and / or degraded.

The present invention is directed to the production of soft gelatin capsules in a conventional manner using gelatin mass having an enteric polymer incorporated into it and to incorporate a mixture containing benzimidazole derivative, and an alkaline reacting substance with larger quantities of hydrophobic oily substance or a mixture of such oily substances into the gelatin shell. The resulting capsules being insoluble up to a pH value of 5.5 in aqueous media, but quickly dissolving above a pH of 6.0.

The invention has been developed based on our finding as a result of sustained R & D work, that the incorporation of benzimidazole derivatives, particularly useful for the treatment of duodenal ulcers, along with an alkaline inert reacting material into a hydrophobic oily substance wherein the benzimidazole derivative is in the form of solution or dispersion, results in extended periods of stability during which period the composition does not get discolored and / or degraded.

In other words, the active ingredient in the composition is kept partially in the form of solution and partially in the form of finely divided particles suspended freely in the oily substance which makes the active ingredient readily absorbable the moment the gastric resistant but intestinal soluble gelatin composition is dissolved.

Such a composition will have an advantage over the existing form of the formulation as the available dosage forms for benzimidazole derivatives are having the total amount of active ingredient in the form of solid particles engulfed in a solid matrix of excipients preferably hydrophilic substances, further coated with protective and gastric resistant enteric polymer coatings. It may take some time to dissolve these coats before the benzimidazole derivative is dissolved into the surronding intestinal fluid and gets absorbed.

Accordingly the main objective of the present invention is to provide an improved pharmaceutical composition containing benzimidazole derivatives having enhanced stability during storage.

According to another objective of the present invention there is provided intestine dissoluble soft gel capsule composition comprising gelatin and an enteric polymer in the form of a free acid or its salt and the pharmaceutical composition comprises benzimidazole derivatives, in particular omeprazole, incorporated in an oily base which is stable during shelf storage.

Still another objective of the invention is to provide a pharmaceutical composition comprising benzimidazole derivatives, to be filled into soft gel capsules, which composition reduces degradation of the benzimidazole derivatives during storage / shelf life.

According to still another objective of the invention there is provided a process for preparation of soft gel capsules comprising benzimidazole derivatives that are resistant to the digestive / gastric juice, a gelatin mass and an enteric polymer in the form of a free acid or as its salt.

Accordingly, the present invention provides, an improved pharmaceutical composition in the form of a soft gel capsule resistant to gastric juice and soluble in intestine useful for the treatment of duodenal ulcers and related ailments which comprises a gelatin shell which is resistant to gastric juice and soluble in intestine having an enteric polymer coating in the form of free acid or its salt, the capsule incorporating a composition comprising of benzimidazole derivative, a hydrophobic oily substance or a mixture of such oily substances, an alkaline inert reacting material, a dispersing agent, a surface active agent and / or a solublising agent; the resulting capsules being insoluble in aqueous medium up to a pH of 5.5 but quickly dissolving above pH of 6.0.,

According to another feature of the present invention, there is provided a process for the preparation of a pharmaceutical composition in the form of a soft gel capsule resistant to gastric juice and soluble in intestine useful for the treatment of duodenal ulcers and related ailments which comprises forming a gelatin shell which is resistant to gastric juice and soluble in intestine having an enteric polymer coating in the form of free acid or its salt, incorporating into the resultant capsule a composition comprising a benzimidazole derivative, a hydrophobic oily substance or a mixture of such oily substances, such substance(s) being insoluble in aqueous medium up to a pH of 5.5 but quickly dissolving above pH of 6.0.,an alkaline inert reacting material, a dispersing agent, a surface active agent and / or a solublising agent.

The capsules so formed are insoluble in aqueous medium up to a pH of 5.5 but quickly dissolve above pH of 6.0.

In a preferred embodiment of the invention, the enteric polymer used in the soft gel capsule composition may be selected from among the polymers but not limited to free acid forms of hydroxypropyl methyl cellulose phthalate, alkylmethacrylate and methacrylic acid ester copolymers, polyvinylacetate phthalate and the like or their ammonia or alkali metal salts. The amount of such enteric polymer employed may range from 5.0 - 40.0 percent, preferably 5.0 - 25.0 percent by weight with reference to the dried shell.

The gelatin mass into which the enteric polymer is incorporated is made up of a composition known in the art and contains gelatin, a plasticizer, preservatives, colourants, opacifiers, flavours etc., as required.

In order to carry out faster dissolution of the enteric polymer for preparing the capsule shell composition, the polymer is first dispersed in water, then an aqueous solution of ammonia or alkali metal salt is mixed while stirring. When alkali metal salt is used it may be selected from substances such as sodium hydroxide, potassium hydroxide, bicarbonate sodium, potassium bicarbonate, sodium carbonate, potassium carbonate etc. The quantity of the base materials used is such that it is sufficient to neutralise 60 to 100 percent of the free acid groups present in the selected enteric polymer.

The excess ammonia or alkali has to be removed from the capsule shell composition to avoid decomposition of the ester couplings in enteric polymers. When aqueous ammonia solution is used to prepare polymer solution, the excess ammonia has to be removed before preparing the capsule after mixing with the gelatin mass, by mixing the mass under reduced pressure in warm condition.

When alkali metal salts are used, the excess alkali is to be neutralized by treating the capsules with an acid selected from any of the following ones, hydrochloric acid, sulphric acid, nitric acid, phosphoric acid, mono carboxylic acids such as acetic acid, propionic acid, benzoic acid etc., dicarboxylic acids such as oxalic acid, maleic acid, fumaric acid etc. The acids are used in the form of cold dilute aqueous solutions in the concentration range of 3 to 30% depending on the type of acid used. The acid treatment may be carried out after manufacturing and partial drying of the capsules to avoid deformation and / or leakage of the capsule contents.

According to another feature of the invention the soft gel capsules are optionally treated with a cross-linking agent that reacts with gelatin and makes it insoluble in gastric juice. The cross-linking agent may be selected from among the aldehydes such as formaldehyde, glutaraldehyde, crotonaldehyde 1,2-phthalic acid aldehyde, 1,3-phthalic acid aldehyde, 1,4-phthalic acid aldehyde or carbodiimides like 1-ethyl-3-[2-morpholinyl-(4)-ethyl]-carbodiimide-metho-p-toluene-sulfonate. The treatment may be done by either coating 0.05 to 1.0% w/v of the substance in an alcohol containing aqueous solution on to the soft gel capsule surface or mixing these substances in the gelatin mass before capsule manufacturing.

According to another feature of the invention the pharmaceutical composition containing benzimidazole derivative, known for its potent proton pump inhibition with powerful inhibitory action against the secretion of gastric juice, is prepared by suspending and/or solubilising the benzimidazole derivative in a carrier mixture composed of a hydrophobic oily carrier material, an alkaline inert reacting material and a dispersing agent and/or a surface active agent. surface active agent. The amount of such benzimidazole derivative used is equivalent to one unit dose recommended depending on the benzimidazole derivative incorporated i.e. for omeprazole the amount incorporated into enteric soft gel capsule may range from 10.0 to 60.0mg per capsule, preferably 20.0 to 40.0 mg per capsule.

The hydrophobic oily material may be selected from among the following fats and oils: Fats and oils of vegetable origin such as sesame oil, corn, maize oil, soybean oil, sunflower oil, arachis oil, gingly oil etc.; animal oils such as fish oil, pig oil, beef oil etc.; esters of straight chained aliphatic oils contained in glycerol such as Sunsoft 700 P-2 (a monoester substance manufactured by Taiho Chemicals Company) Panasete 810 ( a triester substance, manufactured by Nippon Oils and Fats); hydrogenated vegetable oils or a mixture thereof. The amount of such hydrophobic oily material may range from 50.0 to 80.0 percent by weight with reference to the contents filled in a capsule.

The alkaline buffering material present in the pharmaceutical composition may be selected from among but are not restricted to substances such as the sodium, potassium, calcium, magnesium and aluminum salts of phosphoric acid, carbonic acid, citric acid, other suitable organic or inorganic acids; substances used in antacid preparations; meglumine; triethanolamine etc. The amount of such alkaline buffering material present in the composition may range from 5.0 to 40.0 percent, preferably 10.0 to 25.0 percent by weight with reference to the contents filled in capsule.

The substances that increase viscosity of the oily material either by dissolving or by forming a colloidal dispersion are used as dispersing agents. The dispersing agent is selected from among but not restricted to colloidal silicon dioxide, polyvinylpyrrolidone etc. The amount of such suspending agent present in the composition may range from 0.5 to 20.0 percent preferably 1.0 to 10.0 percent by weight with reference to the content filled in capsules.

The surface active agent used as solublising and / or dispersing agents is selected from among but is not restricted to substances such as glyceryl monostearate, polyoxyethylene castor oil derivatives such as Cremophor RH 40, Cremophor EL (Make : BASF Corporation), lecithin, polyoxyethylene sorbitan fatty acid esters, sodium lauryl sulphate, doccusate sodium etc. The amount of such surface active agent present in the composition may range from 2.0 to 20.0 percent preferably 5.0 to 15.0 percent by weight with reference to contents filled in capsule.

The seamless soft gel capsules can be manufactured on a rotary die machine filling with the liquid and / or semi solid composition containing benzimidazole derivatives.

The invention is described in detail in the examples given below which are provided by way of illustration only.

### EXAMPLE - 1

### a) Composition of the Soft gelatin shell:

| **Name of the ingredient** | **Percent by wt.** |
|---|---|
| Gelatin | 35.0 |
| Glycerin | 17.5 |
| Water | 20.0 |
| Hydroxypropyl methyl cellulose phthalate | 7.5 |
| Ammonia solution (25%w/v) | 20.0 |

Gelatin mass is prepared by dispersing gelatin in a mixture of water and glycerin maintained at 70°C. Hydroxypropyl methylcellulose phthalate is dissolved by stirring in to ammonia solution at room temperature. The polymer solution is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / Capsule** |
|---|---|
| Soybean oil | 280.0 |
| Omeprazole | 20.0 |
| Meglumine | 20.0 |
| Lecithin | 30.0 |

Lecithin is dispersed into soybean oil using a mechanical stirrer. Omeprazole and meglumine are added to the dispersion while stirring to obtain a smooth dispersion.

### c) Manufacturing of capsule;

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 2

### a) Composition of the Soft gelatin shell:

| **Name of the ingredient** | **Percent by wt.** |
|---|---|
| Gelatin | 30.0 |
| Glycerin | 15.0 |
| Water | 20.0 |
| Hydroxypropyl methyl cellulose phthalate | 10.0 |
| Ammonia solution (25%w/v) | 25.0 |

Gelatin mass is prepared by dispersing gelatin in a mixture of water and glycerin maintained at 70°C. Hydroxypropyl methyl cellulose phthalate is dissolved by stirring in to ammonia solution at room temperature. The polymer solution is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / Capsule** |
|---|---|
| Soybean oil | 280.0mg |
| Omeprazole | 20.0mg |
| Meglumine | 20.0mg |
| Lecithin | 30.0mg |

Lecithin is dispersed into soybean oil using a mechanical stirrer. Omeprazole and meglumine are added to the dispersion while stirring to obtain a smooth dispersion.

### c) Manufacturing of capsule:

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 3

### a) Composition of the Soft gelatin shell:

| **Name of the ingredient** | **Percent by wt.** |
|---|---|
| Gelatin | 40.0 |
| Glycerin | 17.5 |
| Water | 20.0 |
| Hydroxypropyl methyl cellulose phthalate | 5.0 |
| Ammonia solution (25%w/v) | 17.5 |

Gelatin mass is prepared by dispersing gelatin in a mixture of water and glycerin maintained at 70°C. Hydroxypropyl methyl cellulose phthalate is dissolved by stirring in to ammonia solution at room temperature. The polymer solution is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg** / **Capsule** |
|---|---|
| Soybean oil | 280.0mg |
| Omeprazole | 20.0mg |
| Meglumine | 20.0mg |
| Lecithin | 30.0mg |

Lecithin is dispersed into soybean oil using a mechanical stirrer. Omeprazole and meglumine are added to the dispersion while stirring to obtain a smooth dispersion.

### c) Manufacturing of capsule:

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 4

### a) Composition of the Soft gelatin shell:

| **Name of the ingredient** | **Percent by wt.** |
|---|---|
| Gelatin | 35.0 |
| Glycerin | 17.5 |
| Water | 25.0 |
| Hydroxypropyl methyl cellulose phthalate | 7.5 |
| Ammonia solution (25%w/v) | 15.0 |

Gelatin mass containing hydroxypropyl methyl cellulose is prepared by dispersing hydroxypropyl methyl cellulose phthalate in the form of a fine powder in a mixture of glycerin and water maintained at 70°C in which gelatin is dispersed to dissolve forming the gelatin mass. After cooling the mass to 45°C, ammonia solution is added slowly along the stirrer rod while stirring into the gelatin preparation tank. Stirring is continued till hydroxypropyl methyl cellulose phthalate is completely dissolved. The mass is made bubble free by applying vacuum while maintaining the mass at 45 - 50°C under continuous mixing.

### b) Composition of the medicament:

| **Name of the ingredient** | **mg / capsule** |
|---|---|
| Soybean oil | 200.0mg |
| Cremohor RH 40 | 40.0mg |
| Lansoprazole | 30.0mg |
| Disodium hydrogen orthophosphate Anhydrous | 30.0mg |

Cremophor RH 40 is dispersed in soybean oil at 30°C. After cooling to room temperature Lansoprazole and disodium hydrogen orthophosphate are dispersed in to the mixture in the form of fine particles with the help of a mechanical stirrer and / or a homogeniser.

### c) Manufacturing of capsule:

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 5

### a) Composition of the Soft gelatin shell:

| **Name of the ingredient** | **Percent by wt.** |
|---|---|
| Gelatin | 35.0 |
| Glycerin | 15.0 |
| Water | 20.0 |
| Hydroxypropyl methyl cellulose phthalate | 10.0 |
| Sodium hydroxide solution 1% w/v | 20.0 |

Gelatin mass is prepared by dispersing gelatin in a mixture of water and glycerin maintained at 70°C. Hydroxypropyl methyl cellulose phthalate is dissolved by stirring in to sodium hydroxide solution at room temperature. Hydroxypropyl methyl cellulose phthalate solution in ammonia is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / capsule** |
|---|---|
| Soybean oil | 200.0mg |
| Hydrogenated vegetable oil | 85.0mg |
| Lecithin | 20.0mg |
| Pantoprazole Sodium | 45.0mg |
| Meglumine | 20.0mg |

Hydrogenated vegetable oil is melted and dispersed into soybean oil at 30 - 40°C followed by lecithin, meglumine and pantoprazole sodium and cooled to room temperature. The mixture is kneaded into a smooth paste using a triple roller mill.

### c) Manufacturing of capsule:

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 6

### a) Composition of the Soft gelatin shell:

| **Name of the ingredient** | **Percent by wt.** |
|---|---|
| Gelatin | 30.0 |
| Propylene glycol | 15.0 |
| Water | 20.0 |
| Hydroxypropyl methyl cellulose phthalate | 10.0 |

Gelatin mass is prepared by dispersing in water at 70°C. Hydroxypropyl methyl cellulose phthalate is dissolved in propylene glycol at 60 - 70°C. and mixed with the gelatin mass to obtain uniform mixture.

### b) Composition of the medicament:

| **Name of the ingredient** | **mg / Capsule** |
|---|---|
| Soybean oil | 280.0mg |
| Omeprazole | 20.0mg |
| Meglumine | 20.0mg |
| Lecithin | 30.0mg |

Lecithin is dispersed into soybean oil using a mechanical stirrer. Omeprazole and meglumine are added to the dispersion while stirring to obtain a smooth dispersion.

### c) Manufacturing of capsule:

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 7

### a) Composition of the Soft gelatin shell:

| **Name of the ingredient** | **Percent by wt**. |
|---|---|
| Gelatin | 35.0 |
| Glycerin | 17.5 |
| Water | 20.0 |
| Polyvinylacetate phthalate (PVAP) | 7.5 |
| Ammonia solution (25%/w/v | 20.0 |

Gelatin mass is prepared by dispersing gelatin in a mixture of water and glycerin maintained at 70°C. Polyvinylacetate phthalate is dissolved by stirring into ammonia solution at room temperature. Polyvinylacetate phthalate solution in ammonia is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / capsule** |
|---|---|
| Sunflower oil | 200.0mg |
| Cremophor RH 40 | 40.0mg |
| Lansoprazole | 30.0mg |
| Disodium hydrogen orthophosphate Anhydrous | 30.0mg |

Cremophor RH 40 is dispersed in sunflower oil at 30°C. After cooling to room temperature Lansoprazole and disodium hydrogen orthophosphate are dispersed into the mixture in the form of fine particles with the help of a mechanical stirrer and / or a homogeniser.

### c) Manufacturing of capsule:

This gelatin mixture is transferred to the holding tank of a rotary die capsulation machine for manufacture of a capsule shell. The dispersion containing medicament is transferred to the hopper of the capsulation machine for filling into the soft gel capsules. The soft gel capsules are manufactured by a rotary die process.

### EXAMPLE - 8

### a) Composition of the Soft gelatin shell:

| **Name of the ingredient** | **Percent by wt.** |
|---|---|
| Gelatin | 35.0 |
| Glycerine | 10.0 |
| Triethyl citrate | 7.5 |
| Water | 20.0 |
| Methacrylic acid co-polymer Type - C | 7.5 |
| Ammonia solution (25%w/v) | 20.0 |

Gelatin mass is prepared by dispersing gelatin in a mixture of water triethyl citrate and glycerin maintained at 70°C. Methacrylic acid co-polymer Type - C is dissolved by stirring in to ammonia solution at room temperature. The polymer solution is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / Capsule** |
|---|---|
| Soybean oil | 280.0 |
| Omeprazole | 20.0 |
| Meglumine | 20.0 |
| Colloidal silicon dioxide | 6.0 |

Colloidal silicon dioxide is dispersed into soybean oil using a mechanical stirrer. Omeprazole and meglumine are added to the dispersion while stirring to obtain a smooth dispersion.

### c) Manufacturing of capsule:

This gelatin mixture is transferred to the holding tank of rotary die capsulation machine for manufacture of capsule shell. The dispersion containing medicament is transferred to the hopper of the capsulation machine for filling into the soft gel capsules. The soft gel capsules are manufactured by rotary die process.

### EXAMPLE - 9

### a) Composition of the Soft gelatin shell:

| **Name of the ingredient** | **Percent by wt** |
|---|---|
| Gelatin | 30.0 |
| Glycerin | 15.0 |
| Water | 20.0 |
| Polyvinyl acetate phthalate | 10.0 |
| Ammonia solution (25%w/v) | 25.0 |

Gelatin mass is prepared by dispersing gelatin in a mixture of water and glycerin maintained at 70°C. Polyvinyl acetate phthalate is dissolved by stirring in to ammonia solution at room temperature. The polymer solution is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / Capsule** |
|---|---|
| Sun flower oil | 280.0mg |
| Omeprazole | 20.0mg |
| Meglumine | 20.0mg |
| Lecithin | 30.0mg |

Lecithin is dispersed into Sun flower oil using a mechanical stirrer. Omeprazole and meglumine are added to the dispersion while stirring to obtain a smooth dispersion.

### c) Manufacturing of capsule:

This gelatin mixture is transferred to the holding tank of rotary die capsulation machine for manufacture of capsule shell. The dispersion containing medicament is transferred to the hopper of the capsulation machine for filling into the soft gel capsules. The soft gel capsules are manufactured by rotary die process.

### EXAMPLE - 10

### a) Composition of the Soft gelatin shelf:

| **Name of the ingredient** | **Percent by wt.** |
|---|---|
| Gelatin | 40.0 |
| Triethyl citrate | 7.5 |
| Glycerin | 10.0 |
| Water | 20.0 |
| Methacrylic acid co-polymer Type - A | 7.5 |
| Ammonia solution (25%w/v) | 17.5 |

Gelatin mass is prepared by dispersing gelatin in a mixture of water Triethyl citrate and glycerin maintained at 70°C. Methacrylic acid co-polymer Type - A is dissolved by stirring in to ammonia solution at room temperature. The polymer solution is added to gelatin mass while stirring the mass maintained at 45 - 50°C. Vacuum is applied to the mixing vessel to remove the ammonia evolved and to obtain bubble free transparent mixture of polymer solution and gelatin mass.

### b) Composition of the medicament:

| **Name of the ingredient** | **mg / Capsule** |
|---|---|
| Soybean oil | 280.0mg |
| Omeprazole | 20.0mg |
| Meglumine | 20.0mg |
| Colloidal silicon dioxide | 30.0mg |

Colloidal silicon dioxide is dispersed into soybean oil using a mechanical stirrer. Omeprazole and meglumine are added to the dispersion while stirring to obtain a smooth dispersion.

### c) Manufacturing of capsule:

This gelatin mixture is transferred to the holding tank of rotary die capsulation machine for manufacture of capsule shell. The dispersion containing medicament is transferred to the hopper of the capsulation machine for filling into the soft gel capsules. The soft gel capsules are manufactured by rotary die process.

**The advantages of the present invention are:**
1) Simple method of manufacturing, when compared to the methods disclosed in the prior art making the process economical.
2) Improved bioavailability when compared to the solid enteric coated pellets and tablets as the medicament is solublised or suspended in the form of very fine particles in the liquid / semisolid pharmaceutical composition filled into the soft gel capsule.
3) The reactive acidic groups of enteric polymers are in minimal contact with the active ingredient as the polymer is mixed into large amount of gelatin mass. Only small amounts of alkaline reactive material is required to neutralize the free fatty acids in the oily substances and free acidic reacting groups of enteric polymer in contact with the active ingredient on inner surface of the shell.
4) The soft gel does not require any protective sub-coating. Consequently the active ingredient quickly dissolves into the intestinal fluid once the gastric resistant but intestinal soluble gelatin composition is dissolved.
5) The soft gel capsules are simple in composition and therefore do not require any sophisticated equipment for manufacturing.

## Claims

1. A pharmaceutical composition in form of a soft gel capsule which comprises a gelatin shell having an enteric polymer in the form of a free acid or its salt incorporated into the gelatin, the capsule containing a composition comprising a benzimidazole derivative, a hydrophobic oily substance or a mixture of such hydrophobic oily substances, an alkaline inert reacting material, and a surface active agent and/or a solublising agent; wherein the capsule is insoluble in aqueous medium up to a pH of 5.5 but soluble above a pH of 6.0.

2. A pharmaceutical composition as claimed in claim 1 wherein the benzimidazole derivative is suspended/solubilised in a hydrophobic oily substance selected from fats and oils of vegetable origin; animal origin; esters of straight chain aliphatic oils; hydrogenated vegetable oils or mixtures thereof.

3. A pharmaceutical composition as claimed in claim 1 or claim 2 wherein the hydrophobic oily substance is selected from sesame oil, corn oil, maize oil, soybean oil, sunflower oil, arachis oil, gingly oil, fish oil, pig oil, beef oil, Sunsoft 700 P-2 (Taiho chemical company) and Panasete 810 (Nippon oils and Fats).

4. A pharmaceutical composition as claimed in any preceding claim wherein the enteric polymer is selected from hydroxypropyl methyl cellulose phthalate, alkyl methacrylate and methacrylic acid copolymers, polyvinyl acetate phthalate and the like in the form of free acid or their ammonia or alkali metal salts.

5. A pharmaceutical composition as claimed in any preceding claim wherein the surface active agent and/or a solublising agent is selected from glyceryl monostearate, lecithin, polyoxyethylene castor oil derivatives such as Cremophor RH 40, Cremophor EL (BASF) polyoxyethylene sorbitan fatty acid esters, sodium lauryl sulphate, and docusate sodium.

6. A pharmaceutical composition as claimed in any preceding claim wherein the alkaline inert reacting material is selected from the sodium, potassium, calcium, magnesium and aluminium salts of phosphoric acid, carbonic acid, citric acid, other suitable organic or inorganic acids; substances used in antacid preparations; meglumine; and triethanolamine.

7. A pharmaceutical composition as claimed in any preceding claim wherein the composition further includes a suspending agent.

8. A pharmaceutical composition as claimed in claim 7 wherein the suspending agent is selected from colloidal silicon dioxide, polyvinylpyrrolidone and is present in an amount ranging from 0.5 to 20.0 percent preferably 1.0 to 10.0 percent by weight, with reference to the contents filled in capsule.

9. A pharmaceutical composition as claimed in any preceding claim wherein the soft gel capsules are treated with a gelatin cross linking agent such as formaldehyde, glutaraldehyde, crotonaldehyde, 1,2-phthalic acid aldehyde, 1,3-phthalic acid aldehyde, 1,4-phthalic acid aldehyde; carboimides such as 1-ethyl-3-[2-morpholinyl-(4)-ethyl]-carboimide-metho-P-toluene-sulfonate.

10. A pharmaceutical composition as claimed in any preceding claim wherein the soft gel capsules are treated with cold dilute solutions of acids selected from hydrochloric acid, sulphuric acid, nitric acid, phosphoric acid, citric acid, propionic acid, benzoic acid, oxalic acid, maleic acid, and fumaric acid.

11. A pharmaceutical composition as claimed in any preceding claim wherein the enteric polymer is present in an amount ranging from 5.0 to 40.0 percent, preferably 5.0 to 25.0 percent by weight, with reference to the dried shell.

12. A pharmaceutical composition as claimed in any preceding claim wherein the amount of hydrophobic oily substance ranges from 50.0 to 80.0 percent by weight, with reference to the contents filled in capsules.

13. A pharmaceutical composition as claimed in any preceding claim wherein the alkaline inert reacting material is present in an amount ranging from 5.0 to 40.0 percent, preferably 10.0 to 25.0 percent by weight, with reference to the contents filled in capsule.

14. A pharmaceutical composition as claimed in any preceding claim wherein the surface active agent and/or a solublising agent is present in an amount ranging from 2.0 to 20.0 percent, preferably 5.0 to 15.0 percent by weight, with reference to the contents filled in capsule.

15. A pharmaceutical composition as claimed in any preceding claim wherein the benzimidazole derivative, is selected from omeprazole, lansoprazole, pantoprazole and timoprazole.

## Patentansprüche

1. Pharmazeutische Zusammensetzung in Form einer weichen Gelkapsel, die einen Gelatinemantel mit einem magensaftresistenten Polymer in Form einer freien Säure oder ihres Salzes, eingearbeitet in die Gelatine, umfaßt, wobei die Kapsel eine Zusammensetzung enthält, die ein Benzimidazol-Derivat, eine hydrophobe ölige Substanz oder eine Mischung solcher hydrophoben öligen Substanzen, ein alkalisches inert reagierendes Material und eine oberflächenaktive Substanz und/oder ein Löslichmachungsmittel umfaßt; wobei die Kapsel in wäßrigem Medium bis zu einem pH von 5,5 unlöslich, aber oberhalb eines pHs von 6,0 löslich ist.

2. Pharmazeutische Zusammensetzung nach Anspruch 1, **dadurch gekennzeichnet, daß** das Benzimidazol-Derivat in einer hydrophoben öligen Substanz, die ausgewählt ist aus Fetten und Ölen pflanzlichen Ursprungs; tierischen Ursprungs; Estern geradkettiger aliphatischer Öle; hydrierten pflanzlichen Ölen oder Mischungen davon, suspendiert oder löslich gemacht ist.

3. Pharmazeutische Zusammensetzung nach Anspruch 1 oder Anspruch 2, **dadurch gekennzeichnet, daß** die hydrophobe ölige Substanz ausgewählt ist aus Sesamöl, Maisöl, Maiskeimöl, Sojabohnenöl, Sonnenblumenöl, Arachisöl, Sesamöl aus Sesamum indicum L., Fischöl, Schweineöl, Rinderöl, Sunsoft 700 P-2 (Taiho Chemical Company) und Panasete 810 (Nippon Oils and Fats).

4. Pharmazeutische Zusammensetzung nach irgendeinem vorangehenden Anspruch, **dadurch gekennzeichnet, daß** das magensaftresistente Polymer ausgewählt ist aus Hydroxypropylmethylcellulosephthalat, Alkylmethacrylat und Methacrylsäure-Copolymeren, Polyvinylacetatphthalat und dergleichen in Form von freier Säure oder ihren Ammoniak- oder Alkalimetallsatzen.

5. Pharmazeutische Zusammensetzung nach irgendeinem vorangehenden Anspruch, **dadurch gekennzeichnet, daß** die oberflächenaktive Substanz und/oder ein Löslichmachungsmittel ausgewählt ist aus Glycerylmonostearat, Lecithin, Polyoxyethylen-Rizinusöl-Derivaten, wie etwa Cremophor RH 40, Cremophor EL (BASF), Polyoxyethylensorbitanfettsäureestern, Natriumlaurylsulfat und Docusat-Natrium.

6. Pharmazeutische Zusammensetzung nach irgendeinem vorangehenden Anspruch, **dadurch gekennzeichnet, daß** das alkalische inert reagierende Material ausgewählt ist aus Natrium-, Kalium-, Calcium-, Magnesium- und Aluminiumsalzen von Phosphorsäure, Kohlensäure, Zitronensäure, anderen geeigneten organischen oder anorganischen Säuren; Substanzen, die in säureneutralisierenden Zubereitungen verwendet werden; Meglumin; und Triethanolamin.

7. Pharmazeutische Zusammensetzung nach irgendeinem vorangehenden Anspruch, **dadurch gekennzeichnet, daß** die Zusammensetzung weiter ein Suspensionsmittel einschließt.

8. Pharmazeutische Zusammensetzung nach Anspruch 7, **dadurch gekennzeichnet, daß** das Suspensionsmittel ausgewählt ist aus kolloidalem Siliciumdioxid, Polyvinylpyrrolidon und in einer Menge in einem Bereich von 0,5 bis 20,0 Gew.-%, vorzugsweise 1,0 bis 10,0 Gew.-%, bezogen auf den in die Kapsel gefüllten Inhalt, vorliegt.

9. Pharmazeutische Zusammensetzung nach irgendeinem vorangehenden Anspruch, **dadurch gekennzeichnet, daß** die weichen Gelkapseln mit einem Gelatine-Vernetzungsmittel behandelt werden, wie etwa Formaldehyd, Glutaraldehyd, Crotonaldehyd, 1,2-Phthalsäurealdehyd, 1,3-Phthalsäurealdehyd, 1,4-Phthalsäurealdehyd, Carboimiden wie etwa 1-Ethyl-3-[2-morpholinyl-(4)-ethyl]-carboimid-metho-p-toluolsulfonat.

10. Pharmazeutische Zusammensetzung nach irgendeinem vorangehenden Anspruch, **dadurch gekennzeichnet, daß** die weichen Gelkapseln mit kalten verdünnten Lösungen von Säuren behandelt werden, die ausgewählt sind aus Salzsäure, Schwefelsäure, Salpetersäure, Phosphorsäure, Zitronensäure, Propionsäure, Benzoesäure, Oxalsäure, Maleinsäure und Fumarsäure.

11. Pharmazeutische Zusammensetzung nach irgendeinem vorangehenden Anspruch, **dadurch gekennzeichnet, daß** das magensaftresistente Polymer in einer Menge im Bereich von 5,0 bis 40 Gew.-%, vorzugsweise 5,0 bis 25,0 Gew.-%, bezogen auf den getrockneten Mantel, vorliegt.

12. Pharmazeutische Zusammensetzung nach irgendeinem vorangehenden Anspruch, **dadurch gekennzeichnet, daß** die Menge an hydrophober öliger Substanz von 50,0 bis 80,0 Gew.-%, bezogen auf den in die Kapseln gefüllten Inhalt, reicht.

13. Pharmazeutische Zusammensetzung nach irgendeinem vorangehenden Anspruch, **dadurch gekennzeichnet, daß** das alkalische inert reagierende Material in einer Menge im Bereich von 5,0 bis 40,0 Gew.-%, vorzugsweise 10,0 bis 25,0 Gew.-%, bezogen auf den in die Kapsel gefüllten Inhalt, vorliegt.

14. Pharmazeutische Zusammensetzung nach irgendeinem vorangehenden Anspruch, **dadurch gekennzeichnet, daß** die oberflächenaktive Substanz und/oder ein Löslichmachungsmittel in einer Menge im Bereich von 2,0 bis 20,0 Gew.-%, vorzugsweise 5,0 bis 15,0 Gew.-%, bezogen auf den in die Kapsel gefüllten Inhalt, vorliegt.

15. Pharmazeutische Zusammensetzung nach irgendeinem vorangehenden Anspruch, **dadurch gekennzeichnet, daß** das Benzimidazol-Derivat ausgewählt ist aus Omeprazol, Lansoprazol, Pantoprazol und Timoprazol.

## Revendications

1. Composition pharmaceutique sous la forme d'une capsule de gel souple qui comprend une coque de gélatine comportant un polymère entérique sous la forme d'un acide libre ou de son sel incorporé dans la gélatine, la capsule contenant une composition comprenant un dérivé de benzimidazole, une substance huileuse hydrophobe ou un mélange de ces substances huileuses hydrophobes, un matériau réactionnel alcalin inerte et un agent tensioactif et/ou un agent de solubilisation ; la capsule étant insoluble dans un milieu aqueux jusqu'à un pH de 5,5 mais soluble au-delà d'un pH de 6,0.

2. Composition pharmaceutique selon la revendication 1, dans laquelle le dérivé de benzimidazole est mis en suspension/solubilisé dans une substance huileuse hydrophobe choisie parmi des matières grasses et des huiles d'origine végétale ; d'origine animale ; des esters d'huiles à chaîne linéaire aliphatiques ; des huiles végétales hydrogénées ou leurs mélanges.

3. Composition pharmaceutique selon la revendication 1 ou la revendication 2, dans laquelle la substance huileuse hydrophobe est choisie parmi l'huile de sésame, l'huile de blé, l'huile de maïs, l'huile de soja, l'huile de tournesol, l'huile d'arachide, l'huile de gingly, l'huile de poisson, la graisse de porc, la graisse de boeuf, le Sunsoft 700 P-2 (Taiho chemical compagny) et le Panasete 810 (Nippon oils and Fats).

4. Composition pharmaceutique selon l'une quelconque des revendications précédentes dans laquelle le polymère entérique est choisi parmi l'hydroxypropyl-méthylcellulose phtalate, l'alkyle méthacrylate et des copolymères d'acide méthacrylique, l'acétate phtalate de polyvinyle, et autres, sous la forme d'acide libre ou de leurs sels d'ammonium ou sels de métal alcalin.

5. Composition pharmaceutique selon l'une quelconque des revendications précédentes dans laquelle l'agent tensioactif et/ou l'agent de solubilisation est choisi parmi le monostéarate de glycéryle, la lécithine, les dérivés polyoxyéthylène d'huile de ricin tels que le Cremophor RH 40, le Cremophor EL (BASF), des esters d'acide gras de polyoxoéthylène sorbitan, le lauryle sulfate de sodium et le docusate de sodium.

6. Composition pharmaceutique selon l'une quelconque des revendications précédentes dans laquelle le matériau réactionnel alcalin inerte est choisi parmi les sels de sodium, de potassium, de calcium, de magnésium et d'aluminium d'acide phosphorique, d'acide carbonique, d'acide citrique, d'autres acides organiques ou inorganiques appropriés ; des substances utilisées dans des préparations anti-acides ; la méglumine et la triéthanolamine.

7. Composition pharmaceutique selon l'une quelconque des revendications précédentes dans laquelle la composition comprend en outre un agent de suspension.

8. Composition pharmaceutique selon la revendication 7, dans laquelle l'agent de suspension est choisi parmi le dioxyde de silicium colloïdal, la polyvinylpyrrolidone et est présent en une quantité de l'ordre de 0,5 à 20,0 pour cent, de préférence de 1,0 à 10,0 pour cent en poids, en référence aux contenus remplissant la capsule.

9. Composition pharmaceutique selon l'une quelconque des revendications précédentes dans laquelle les capsules de gel souples sont traitées avec un agent de réticulation de la gélatine tel que le formaldéhyde, le glutaraldéhyde, le crotonaldéhyde, l'aldéhyde d'acide 1,2-phtalique, l'aldéhyde d'acide 1,3-phtalique, l'aldéhyde d'acide 1,4-phtalique ; des carbo-imides tels que le 1-éthyl-3[2-morpholinyl-(4)éthyl] carbo-imide-métho-P-toluène-sulfonate.

10. Composition pharmaceutique selon l'une quelconque des revendications précédentes dans laquelle les capsules de gel souples sont traitées avec des solutions diluées froides d'acides choisis parmi l'acide chlorhydrique, l'acide sulfurique, l'acide nitrique, l'acide phosphorique, l'acide citrique, l'acide propionique, l'acide benzoïque, l'acide oxalique, l'acide maléique et l'acide fumarique.

11. Composition pharmaceutique selon l'une quelconque des revendications précédentes dans laquelle le polymère entérique est présent en une quantité de l'ordre de 5,0 à 40,0 pour cent, de préférence de 5,0 à 25,0 pour cent en poids, en référence à la coque sèche.

12. Composition pharmaceutique selon l'une quelconque des revendications précédentes dans laquelle la quantité de substance huileuse hydrophobe est de l'ordre de 50,0 à 80,0 pour cent en poids, en référence aux contenus remplissant les capsules.

13. Composition pharmaceutique selon l'une quelconque des revendications précédentes dans laquelle le matériau réactionnel alcalin inerte est présent en une quantité de l'ordre de 5,0 à 40,0 pour cent, de préférence de 10,0 à 25,0 pour cent en poids, en référence aux contenus remplissant la capsule.

14. Composition pharmaceutique selon l'une quelconque des revendications précédentes dans laquelle l'agent tensioactif et/ou l'agent de solubilisation est présent en une quantité de l'ordre de 2,0 à 20,0 pour cent, de préférence de 5,0 à 15,0 pour cent en poids, en référence aux contenus remplissant la capsule.

15. Composition pharmaceutique selon l'une quelconque des revendications précédentes dans laquelle le dérivé de benzimidazole est choisi parmi l'oméprazole, le lansoprazole, le pantoprazole et le timoprazole.
